# EUROPEAN PATENT APPLICATION

(11) **EP 1 666 441 A1**
(43) Date of publication of application: **07.06.2006**
(21) Application number: 05257406.8
(22) Date of filing: 01.12.2005
(51) Int. Cl.: C07B 41/06, C07C 45/30, C07C 47/02, C07C 47/54, C07C 47/228, C07C 47/575, C07C 49/04, C07C 49/403

(54) **Oxidation method for primary or secondary alcohols**

(30) Priority: 03.12.2004 JP 2004350780
(71) Applicant: DAISO CO., LTD., Osaka-shi Osaka-fu (JP)
(72) Inventor: Igi, Kimitaka c/o Daiso Co., Ltd., Osaka-shi, Osaka-fu (JP); Hirata, Makoto c/o Daiso Co., Ltd., Osaka-shi, Osaka-fu (JP); Mikami, Masafumi c/o Daiso Co., Ltd., Osaka-shi, Osaka-fu (JP)
(74) Representative: Stoner, Gerard Patrick

(57) **Abstract**

A method for preparing an aldehyde or ketone by oxidizing a primary or secondary alcohol in the presence of a nitroxyl radical compound and a co-oxidant in an organic solvent, which process is characterized in using an organic N-bromoamide compound or a combination of N-chlorosuccinimide and a compound having bromide ion as the co-oxidant.

## Description

### TECHNICAL FIELD

The present invention relates to a novel method for preparing an aldehyde or ketone by oxidizing a primary or secondary alcohol.

### BACKGROUND

The oxidation of alcohols into aldehydes or ketones is one of the important methods in organic synthesis. It is known that alcohols can be oxidized with sodium hypochlorite as an oxidizing agent in the presence of catalytic amount of 2,2,6,6-tetramethyl-1-piperidine-1-oxyl radical (abbreviated as TEMPO hereinafter) (See J. Org. Chem., 1987, 52, p.2529). Furthermore, it is reported that a N-chloro compound such as N-chlorosuccinimide (NCS) or trichloroisocyanuric acid can be used as an oxidizing agent (See US Patent 5821374, and J. Org. Chem., 1996, 61, p.7452).

The present inventors have studied extensively and found that by using a N-bromoamide compound or a combination of NCS and a compound having bromide ion, oxidation of alcohols effectively proceeds in the presence of a TEMPO-type compound derivative as catalyst in an organic solvent.

The present invention provides novel methods for effectively preparing an aldehyde or ketone by oxidizing a primary or secondary alcohol in an organic solvent.

Namely, the present invention relates to the method for preparing an aldehyde or ketone by oxidizing a primary or secondary alcohol in the presence of a nitroxyl radical compound represented by the following formula (1), 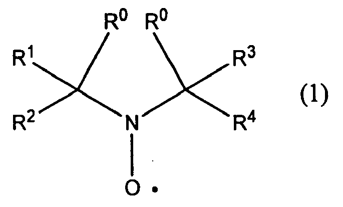 wherein R⁰ to R⁴ are the same or different and straight or branched C₁₋₁₀ alkyl group, or two R⁰s may be combined together with the nitrogen atom in the intramolecule to form a 5 to 7 membered hetero ring,
and a co-oxidant in an organic solvent,
which process is characterized in using an organic N-bromoamide compound or a combination of N-chlorosuccinimide and a compound having bromide ion as said co-oxidant.

As the present process can be carried out in an organic solvent, troublesome adjustment of pH can be avoided and the degradation of the product is found hardly to occur. As a brominated compound is used as a co-oxidant, opportunities to use this reaction increase.

Although the catalyst used in the present invention is a catalyst represented by the above formula (1), it may include such a compound wherein at least one of R⁰ to R⁴ represents C₁₋₆ alkyl group substituted by C₁₋₆ alkoxy group in the formula (1), as long as it is effective in the process.

Catalysts of formula (1) are known and may e.g. be prepared by the methods described in European Patents 574666 and 574667.

As the catalyst (1) a compound of the following formula (1a) is preferable, 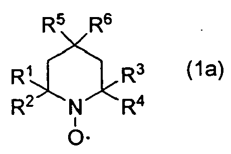 wherein R¹ to R⁴ are the same as defined above, R⁵ and R⁶ are both hydrogen atom or both C₁₋₆ alkoxy group, or one of R⁵ and R⁶ is hydrogen atom and the other is hydroxy group, C₁₋₆ alkoxy group, acyloxy group, or acylamino group, or R⁵ and R⁶ taken together may form any one of ketal groups of the formulas (a) to (c), 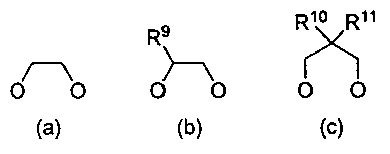 wherein R⁹ is C₁₋₆ alkyl, and R¹⁰ and R¹¹ are H or C₁₋₆ alkyl, e.g. both hydrogen atom or the same or different C₁₋₆ alkyl groups.

The compound (1a) wherein R¹ to R⁴ are all methyl group, and R⁵ and R⁶ are both hydrogen atom, or one of R⁵ and R⁶ is hydrogen atom and the other is hydroxy group, methoxy group, acetoxy group, benzoyloxy group or acetamide group is especially preferable.

The amount of the catalyst (1) or (1a) is preferably 0.01 to 30 mole % against the substrate (alcohol), especially preferably 0.05 to 5 mole %.

The preferable organic N-bromoamide compounds used as a co-oxidant in this invention include N-bromoacetamide (NBA), N-bromosuccinimide (NBS), tribromoisocyanuric acid, dibromo dimethyl hydantoin, N-bromophthalimide and so on. NBA and NBS among them are especially preferable.

When a combination of N-chlorosuccinimide and a compound having bromide ion is used as a co-oxidant, an alkali metal bromide such as sodium bromide or potassium bromide, and a quaternary ammonium bromide such as tetrabutylammonium bromide or benzyltrimethylammonium bromide are preferably used as a compound having bromide ion (a source of said bromide ion, or ionic bromide compound).

When an organic N-bromoamide compound is used as a co-oxidant, the preferred amount of the organic N-bromoamide compound is 1.05 to 2 molar equivalents against the substrate (alcohol), and more preferably 1.1 to 1.5 molar equivalents.

On the other hand, when a combination of N-chlorosuccinimide and a compound having bromide ion is used as a co-oxidant, the amount of N-chlorosuccinimide is preferably 1.05 to 2 molar equivalents against a substrate, an alcohol and more preferably 1.1 to 1.5 molar equivalents. The amount of the compound having bromide ion is preferably 1 to 100 mole % against the substrate (alcohol) and more preferably 5 to 20 mole %.

The alcohol used as a substrate in the present invention is not limited as long as it has an alcoholic primary or secondary hydroxy group, for example, a straight or branched primary or secondary alkyl alcohol such as 1-octanol, 1-nonanol or 2-octanol, a cycloalkyl alcohol such as cyclohexanol or cyclobutanol, or an alcohol having substituted or unsubstituted aralkyl group such as phenethyl alcohol, benzyl alcohol or p-methoxybenzyl alcohol. Furthermore, glycerol acetonide is also preferably used.

It is not necessary to use water in the present process. In order to adjust the viscosity a suitable organic solvent may be used if necessary, for example an ether-solvent such as tetrahydrofuran (THF), diethyl ether, 1,2-diethoxyethane or methyl t-butyl ether (MTBE), an ester-solvent such as ethyl acetate or butyl acetate, an aromatic hydrocarbon-solvent such as benzene or toluene, a hydrocarbon-solvent such as hexane or heptane, a ketone-solvent such as acetone or methylethylketone, a halogeno compound-solvent such as dichloromethane or 1,2-dichloroethane, or a tertiary alcohol such as t-butanol or t-amyl alcohol. The ester-solvent, the ketone-solvent and the halogeno compound-solvent are preferably used.

The reaction temperature is not critical, and preferably, is -50 to 100°C and more preferably -15 to 30°C.

In order to neutralize hydrogen bromide occurring in the oxidation reaction, a base may be used such as preferably an inorganic base such as sodium acetate, sodium hydrogencarbonate, potassium carbonate, sodium phosphate or potassium hydrogenphosphate, or an organic base such as triethylamine, diisopropylethylamine or pyridine. When the aldehyde produced is unstable, the aldehyde may be converted into a stable compound by reacting it with a suitable compound.

When an optically active alcohol as a substrate is used, the remarkable racemization does not occur by the oxidation reaction of the present invention and the object aldehyde compound with the optical configuration is obtainable.

### Example

The present invention is explained by the following examples, but the scope of the present invention should not be limited by them. In each example, the product produced was confirmed by showing the same retention time as the standard by gas chromatography.

### Example 1

### Manufacture of 1-octanal

1-Octanol (2.0g, 15.4mmol), sodium hydrogencarbonate (1.6g, 18.5mmol), TEMPO (24mg, 0.15mmol) and dichloromethane (15ml) were put in a 50-ml egg plant type flask. The suspension was cooled to less than 10°C in an ice bath, and thereto was added NBS (3.0g, 16.9mmol) divided in three portions. The insoluble materials were filtered off and the filtrate was washed with 5% aqueous sodium bicarbonate solution. The crude product was purified by distillation to give 1-octanal (1.74g, yield 88%).

### Example 2

### Manufacture of 1-octanal

1-Octanol (2.0g, 15.4mmol), sodium acetate (1.8g, 21.6mmol), 4-hydroxy TEMPO (53mg, 0.31mmol) and dichloromethane (15ml) were put in a 50-ml egg plant type flask. The suspension was cooled to less than 10 °C in an ice bath, and thereto was added NBA (2.5g, 18.5mmol)divided in two portions. The insoluble materials were filtered off and the filtrate was washed with 5% aqueous sodium bicarbonate solution. The crude product was purified by distillation to give 1-octanal (1.78g, yield 90%).

### Example 3

### Manufacture of 1-octanal

1-Octanol (2.0g, 15.4mmol), sodium hydrogencarbonate (1.6g, 18.5mmol), 4-methoxy TEMPO (29mg, 0.15mmol), sodium bromide (0.16g, 1.54mmol) and 1,2-dichloroethane (15ml) were put in a 50-ml egg plant type flask. The suspension was cooled to less than 10°C in an ice bath and thereto was added NCS (2.3g, 16.9mmol) divided in two portions. The insoluble materials were filtered off and the filtrate was washed with 5% aqueous sodium bicarbonate solution. The crude product was purified by distillation to give 1-octanal (1.67g, yield 85%).

### Example 4

### Manufacture of 1-hexanal

1-Hexanol (2.0g, 19.6mmol), sodium carbonate (1.2g, 11.8mmol), 4-acetoamino TEMPO (42mg, 0.20mmol) and dichloromethane (15ml) were put in a 50-ml egg plant type flask. The suspension was cooled to less than in an ice bath and thereto was added NBS (3.8g, 21.6mmol) divided in four portions. The insoluble materials were filtered off and the filtrate was washed with 5% aqueous sodium bicarbonate solution. The crude product was purified by distillation to give 1-hexanal (1.77g, yield 90%).

### Example 5

### Manufacture of phenylacetaldehyde

β-Phenethylalcohol (2.0g, 16.4mmol), sodium acetate (2.0g, 24.6mmol), TEMPO (26mg, 0.16mmol) and ethyl acetate (15ml) were put in a 50-ml egg plant type flask. The suspension was cooled to less than 10°C in an ice bath, and thereto was added NBS (3.2g, 18.0mmol) divided in two portions. The insoluble materials were filtered off and the filtrate was washed with 5% aqueous sodium bicarbonate solution. The crude product was purified by distillation to give phenethylacetaldehyde (1.67g, yield 85%).

### Example 6

### Manufacture of 2-octanone

2-Octanol (2.0g, 15.4mmol), sodium hydrogencarbonate (1.6g, 18.5mmol), TEMPO (24mg, 0.15mmol) and 1,2-dichloroethane (15ml) were put in a 50-ml egg plant type flask. The suspension was cooled to less than 10°C in an ice water bath, and thereto was added NBS (3.0g, 16.9mmol) divided in two portions. The insoluble materials were filtered off and the filtrate was washed with 5% aqueous sodium bicarbonate solution. The crude product was purified by distillation to give 2-octanone (1.88g, yield 95%).

### Example 7

### Manufacture of cyclohexanone

Cyclohexanol (2.0g, 20mmol), sodium carbonate (1.3g, 12mmol), 4-acetoaminoTEMPO (43mg, 0.2mmol) and THF (15ml) were put in a 50-ml egg plant type flask. To the suspension was added NBS (3.9g, 22mmol) divided in two portions. The insoluble materials were filtered off and the filtrate was washed with 5% aqueous sodium bicarbonate solution. The crude product was purified by distillation to give cyclohexanone (1.73g, yield 88%).

### Example 8

### Manufacture of benzaldehyde

Benzyl alcohol (2.0g, 18.5mmol), potassium carbonate (1.5g, 11.1mmol), 4-hydroxy TEMPO (31mg, 0.19mmol), tetrabutylammonium bromide (0.5g, 1.9mmol) and MTBE (15ml) were put in a 50-ml egg plant type flask. The suspension was cooled to less 10°C in an ice bath and thereto was added NCS (2.7g, 20.4mmol) divided in three portions. The insoluble materials were filtered off and the filtrate was washed with 5% aqueous sodium bicarbonate solution. The crude product was purified by distillation to give benzaldehyde (1.77g, yield 90%).

### Example 9

### Manufacture of p-anisaldehyde

p-Methoxybenzyl alcohol (2.0g, 14.5mmol), sodium carbonate (0.9g, 8.7mmol), TEMPO (23mg, 0.15mmol), benzyltrimethylammonium bromide (0.3g, 1.5mmol) and dichloromethane (15ml) were put in a 50-ml egg plant type flask. The suspension was cooled to less than 10°C in an ice bath and thereto was added NCS (2.1g, 16mmol) divided in two portions. The insoluble materials were filtered off and the filtrate was washed with 5% aqueous sodium bicarbonate solution. The crude product was purified by distillation to give p-anisaldehyde (1.62g, yield 82%).

### Example 10

### Manufacture of (R)-3-(2,2-dimethyl-1,3-dioxolan-4-yl)-2-propenoic acid methyl ester

(R)-Glycerol acetonide (5.0g, 37.8mmol), sodium hydrogencarbonate (4.8g, 56.7mmol), 4-acetoxy TEMPO (41mg, 0.19mmol) and THF (50ml) were put in a 200-ml three neck-flask. The suspension was cooled to less than 10°C, and thereto was added NBS (8.1g, 45.4mmol) divided in three portions. The insoluble materials are removed by filtration and the filtrate was cooled to 0°C. Thereto was added methyl (triphenylphosphoranylidene)acetate (15.1g, 45.4mmol) and the mixture was stirred at 0°C for 16 hours. To the reaction mixture was added hexane (100ml) and the insoluble materials were filtered off and the filtrate was concentrated. The crude product was purified by silica gel chromatography to give the object ester (trans form/cis form =29/71) (5.3g, yield 75%).

The present invention relates to a method for preparing an aldehyde or a ketone by oxidizing a primary or secondary alcohol. The compound thus prepared is utilized in the organic synthesis-industry.

It should not need to be stated that where the upper and lower limits of stated ranges are governed by distinct technical criteria, as they generally are, then those upper and lower limits can be treated independently of one another.

## Claims

1. A method for preparing an aldehyde or ketone by oxidizing a primary or secondary alcohol in the presence of a nitroxyl radical compound represented by the following formula (1), 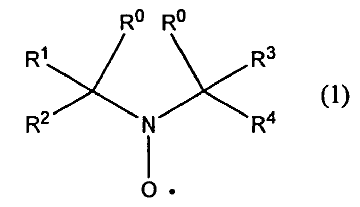 wherein R⁰ to R⁴ are the same or different and are straight or branched C₁₋₁₀ alkyl group, or two R⁰s may be combined together with the nitrogen atom in the molecule to form a 5 to 7 membered hetero ring,
and a co-oxidant, in an organic solvent,
which process is **characterized by** using organic N-bromoamide compound, or a combination of N-chlorosuccinimide and a compound having bromide ion, as said co-oxidant.

2. A process of claim 1 wherein the nitroxyl radical compound is a compound of the following formula (1a), 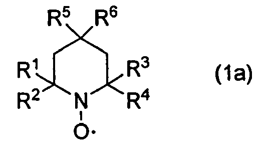 wherein R¹ to R⁴ are the same as defined above, R⁵ and R⁶ are both hydrogen atom or both C₁₋₆ alkoxy group, or one of R⁵ and R⁶ is hydrogen atom and the other is hydroxy group, C₁₋₆ alkoxy group, acyloxy group or acylamino group, or R⁵ and R⁶ taken together may form any one of ketal groups of the formulas (a) to (c), 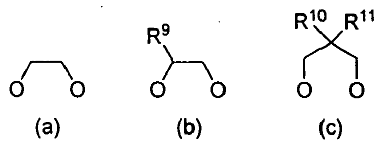 wherein R⁹ is C₁₋₆ alkyl, and R¹⁰ and R¹¹ are both hydrogen atom or the same or different and are C₁₋₆ alkyl group.

3. A process of claim 2 wherein the nitroxyl radical compound is a compound of the formula (1a) wherein R¹ to R⁴ are all methyl group, and R⁵ and R⁶ are both hydrogen atom, or one of R⁵ and R⁶ is hydrogen atom and the other is hydroxy group, methoxy group, acetoxy group, benzoyloxy group or acetamide group.

4. A process of any one of claims 1 to 3 wherein the co-oxidant is an organic N-bromoamide compound.

5. A process of claim 4 wherein the organic N-bromoamide compound is N-bromosuccinimide or N-bromoacetamide.

6. A process of any one of claims 1 to 3 wherein the co-oxidant is a combination of N-chlorosuccinimide and a compound having bromide ion.

7. A process of claim 6 wherein the compound having bromide ion (the source of bromide ion) is an alkali metal bromide or a quaternary ammonium bromide.

8. A process of claim 6 wherein the compound having bromide ion (the source of bromide ion) is sodium bromide or potassium bromide.
